# EUROPEAN PATENT SPECIFICATION

(11) **EP 1 196 144 B1**
(45) Date of publication and mention of the grant of the patent: **22.09.2004**
(21) Application number: 00947430.5
(22) Date of filing: 14.07.2000
(51) Int. Cl.: A61K 9/127

(54) **A LIPOSOME COMPOSITION HAVING RESISTANCE TO FREEZE/THAW DAMAGE**
GEGEN GEFRIER/AUFTAU-SCHADEN RESISTENTE LIPOSOMZUSAMMENSETZUNG
COMPOSITION DE LIPOSOME RESISTANTE AUX DOMMAGES DUS A LA CONGELATION/DECONGELATION

(30) Priority: 16.07.1999 US 144380 P
(43) Date of publication of application: 17.04.2002
(73) Proprietor: Alza Corporation, Mountain View, CA 94039-7210 (US)
(72) Inventor: ABRA, Robert, M., San Francisco, CA 94112 (US); DIBBLE, Andrew, R., Vista, CA 92083 (US)
(74) Representative: Williams, Paul Edwin
(86) International application number: PCT/US2000/019393
(87) International publication number: WO 2001/005372

(56) References cited:
- US-A- 4 883 665
- KOICHIRO MIYAJIMA, ET AL.: "Effect of Saccharides on the Freezing and Thawing of Liposome Dispersion" CHEMICAL AND PHARMACEUTICAL BULLETIN, vol. 34, no. 7, July 1986 (1986-07), pages 2689-2697, XP002157891

## Description

### Field of the Invention

The present invention relates to a liposome composition having improved protection against vesicle aggregation, vesicle fusion, and loss of entrapped contents upon freezing and subsequent thawing.

### Cross Reference to Related Applications

The complete disclosure set forth in the U.S. provisional patent application entitled "Cryoprotection of a Liposome Composition," Serial No.: 60/144,380, filed with the United States Patent and Trademark Office on July 16, 1999, is incorporated herein. The applications are commonly owned.

### Background of the Invention

Liposomes are closed lipid vesicles used for a variety of purposes. In particular, liposomes may be employed to carry therapeutic agents to a target region or cell by systemic administration of the liposomes. Liposomes have proven particularly valuable to buffer drug toxicity and to alter pharmacokinetic parameters of certain therapeutic compounds, for example, doxorubicin, and amphotericin B. Products that incorporate these compounds are commercially available.

The stability and effective storage of pharmaceutical liposome preparations is an important aspect of liposome products. Specifically, it is important that liposome preparations be stored for extended periods of time under appropriate conditions without an undue loss of the encapsulated agent or alteration in size of the liposomes. A concern, however, is that when liposomes are dehydrated or are frozen and subsequently thawed, vesicle fusion and/or leakage of the entrapped contents may occur.

A common method to protect vesicle integrity during dehydration and freezing is to include a cryoprotectant, such as a sugar, in the liposome formulation (Harrigan et al., Chemistry and Physics of Lipids, 52:139-149 (1990)). The cryoprotectant preserves the integrity of the lipids and prevents vesicle fusion and loss of vesicle contents.

U.S. Patent No. 4,927,571, for example, relates to a liposome composition containing doxorubicin which is reconstituted from a lyophilized form that includes between 1 percent (%) and 10% of a cryoprotectant, such as trehalose or lactose.

U.S. Patent No. 4,880,635 relates to a dehydrated liposome composition that is prepared by drying the liposomes in the presence of a sugar, wherein the sugar is present on both the inside and outside surface of the liposome bilayer membrane. Similarly, U.S. Patent No. 5,077,056 relates to a dehydrated liposome composition which includes a protective sugar, preferably on both the internal and external liposome surfaces.

KOICHIRO MIYAJIMA, et al.; 'Effect of Saccharides on the Freezing and Thawing of Liposome Dispersion, CHEMICAL AND PHARMACEUTICAL BULLETIN, vol. 34, no. 7, July 1986, pages 2689 - 2697 discloses a liposome composition comprising aqueous glucose solutions in both the inner and outer phases, wherein the composition is stable to freezing at -70°C.

Other liposome formulations, such as DOXIL®, a liposomal formulation containing doxorubicin, are suspensions wherein the liposomes are not dehydrated for later reconstitution, but remain in a suspension during storage. The suspension medium, however, typically includes a sugar for protection from freezing damage.

### Summary of the Invention

Accordingly, an aspect of the present invention is to provide a liposome composition that is protected from vesicle aggregation and fusion, and protects against a loss of entrapped contents upon freezing and subsequent thawing.

In another aspect, the present invention provides a liposome composition having improved protection from freeze/thaw damage, as evidenced by a reduction in vesicle fusion and a reduction in the loss of entrapped contents, when compared to the protection provided by the presence of a cryoprotectant alone.

The present invention further provides a liposome composition having an entrapped drug with a high ionic strength that is protected from vesicle fusion and loss of contents upon freezing and subsequent thawing.

In yet another aspect of the invention, a liposome composition is provided having improved protection from freeze/thaw damage. In one embodiment, the composition is composed of a suspension of liposomes, each liposome having an entrapped aqueous medium having an inner osmolarity. The liposomes are suspended in an external medium composed of a water soluble salt and a cryoprotectant, wherein the external medium has an outer osmolarity higher than the inner liposome osmolarity thereby establishing a lower inside/higher outside osmotic gradient across each liposome.

In another embodiment, the external medium includes a salt selected from nitrate salts, sodium salts, potassium salts, ammonium salts, and sulfate salts. In a preferred embodiment, the salt is sodium chloride (NaCl) or potassium chloride (KCl).

The cryoprotectant is typically a sugar, either a monosaccharide or a disaccharide, glycerol, or polyethyleneglycol. In a preferred embodiment, the cryoprotectant is selected from trehalose, maltose, sucrose, glucose, lactose, dextran, glycerol, polyethyleneglycol, and aminoglycosides.

In another embodiment, the composition further includes a therapeutic agent entrapped in the liposomes. In a preferred embodiment, the therapeutic agent is cisplatin, or a cisplatin analogue, or derivative thereof.

In still another embodiment, the liposome composition includes a vesicle-forming lipid derivatized with a hydrophilic polymer chain.

In another aspect, the invention provides a method for protecting a liposome composition from freeze/thaw damage. The method includes preparing a suspension of liposomes, each liposome in the suspension having an entrapped aqueous medium having an inner osmolarity. The liposomes are suspended in an external medium composed of a water soluble salt and a cryoprotectant, wherein the external medium has an outer osmolarity higher than the inner liposome osmolarity thereby establishing a lower inside/higher outside osmotic gradient across each liposome.

In still another aspect, the invention provides a method for protecting a liposome composition containing a metal coordination compound against freeze/thaw damage. The method includes preparing a suspension of liposomes, each liposome in the suspension having an entrapped aqueous medium having an inner osmolarity and an entrapped compound. The liposomes are suspended in an external medium composed of a water soluble salt and a cryoprotectant, wherein the external medium has an outer osmolarity higher than the inner liposome osmolarity thereby establishing a lower inside/higher outside osmotic gradient across each liposome. In one embodiment, the therapeutic agent is cisplatin, or a cisplatin analogue or derivative thereof. In another embodiment, the salt in the external medium is sodium chloride (NaCl) and the cryoprotectant is sucrose.

In yet another aspect, the invention provides a method for improving cryoprotection of a suspension of liposomes that includes a cryoprotectant in the suspension medium. The improvement includes a water soluble salt in the suspension medium effective to establish an osmotic gradient across each liposome, wherein the suspension medium has a higher osmolarity than the liposome interior. In one embodiment, the osmotic gradient is at least about 100 mOsm, preferably greater than about 200 mOsm, more preferably greater than about 400 mOsm, and most preferably greater than about 500 mOsm.

### Detailed Description of the Invention

### I. Definitions

As used herein, the following terms have the following meanings:
"Freezing damage" or "freeze damage" refers to any one of several undesirable effects that occur upon the exposure of a liposome composition to a temperature sufficient to cause one of the undesirable effects. A sufficient temperature that results in freezing damage is typically a temperature lower than about 0°C, more typically a temperature lower than about -5°C, and even more typically a temperature lower than about -10°C. Undesirable effects include, but are not limited to, an increase in particle size growth due to aggregation and/or fusion of vesicles, and loss of an encapsulated therapeutic agent. The actual temperature which can cause onset of such an effect will vary according to the liposome formulation, e.g., the type of lipids and other bilayer components, as well as the entrapped medium and therapeutic agent. Sometimes freezing damage is less at very cold temperatures, particularly if the rate of freezing and subsequent thawing is rapid.
"Freeze/thaw damage," as used herein, includes the undesirable side effects that are associated with freezing damage (described above), and additional effects observed as a result of at least one freezing and thawing cycle, e.g., a non-homogeneous particle size distribution. Described effects may be more pronounced during a slow thawing process, i.e., at about 2-8°C, versus a more rapid thawing process, i.e., at room temperature, or exposure to a water bath. (See, for example, Table 8).
"Cryoprotectant" refers to an agent or compound suitable to protect a liposome composition from freezing damage and/or freeze/thaw damage. Preferred cryoprotectants include, for example; sugars (disaccharides and monosaccharides), glycerol, and polyethylene glycol.
"Osmotic gradient" values, as reported herein, were determined by calculating inner and outer osmolarity of the liposome composition, taken as the molarity times one (M x 1) for a nonelectrolyte or the number of ions per molecule for an electrolyte, in both the inner and outer liposome media and subtracting the inner osmolarity from the outer osmolarity.

### II. Exemplary Liposome Compositions

As described above, the present invention relates to liposome compositions exhibiting resistance to freezing damage and/or freeze/thaw damage. The liposome compositions have an osmotic gradient across the liposome lipid bilayer, wherein the suspension medium external to the liposome has a higher osmolarity than the osmolarity of the medium entrapped within the liposome.

For example, Example 1 (below) describes the preparation of two placebo liposome formulations, i.e., one liposome formulation having the cryoprotectant sucrose in both the internal and external liposome media, and the other liposome formulation having sucrose present in the external medium only. Both liposome formulations had 0.9% weight/volume (w/v) NaCl present in the liposome interior and exterior media, and the liposomes were composed of the lipids HSPC, cholesterol, and mPEG-DSPE in a molar ratio of 51/44/5, respectively.

The two liposome formulations were frozen at -20°C for 2.7 days (approximately 65 hours) and subsequently thawed by resting at room temperature. Dynamic light scattering was performed on the two placebo liposome formulations to determine particle size before and after freezing. The results are shown in Table 1.

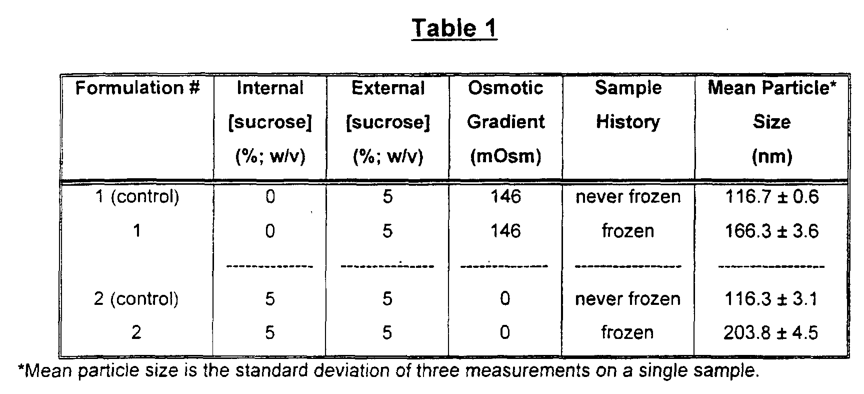

The liposome formulation that had sucrose present in the internal and external media (formulation #2), was more susceptible to freezing damage as evidenced by the increase in particle size, i.e., from approximately 116 nm (the never frozen control) to 204 nm for the freeze/thaw sample. The liposome formulation containing sucrose in the external medium alone (formulation #1), yielded a smaller particle size increase after freezing to approximately 166 nm. As will be more fully demonstrated in the Examples below, these results suggest that a liposome formulation having a hyperosmotic gradient, i.e., an external osmolarity greater than internal osmolarity, (e.g., formulation #1 which has an osmotic gradient of 146 mOsm), was less susceptible to freeze/thaw damage.

Example 2 describes the preparation of a placebo liposome formulation with the above-described lipid composition containing 5% (w/v) sucrose externally and containing no sucrose internally. The liposome formulation was subsequently diluted with 5% (w/v) sucrose and varying amounts of NaCl to yield liposome formulations having 0.9%, 0.6%, and 0.3% (w/v) NaCl in the external aqueous medium. The NaCl concentration in the internal aqueous medium was 0.9% (w/v). Samples of each liposome formulation were frozen at -20°C for approximately 5 days (approximately 118 hours), and subsequently thawed. Particle size was determined, and the results are shown below in Table 2.

**Table 2**

| **External [NaCl] (%; w/v)** | **External [sucrose] (%; w/v)** | **Osmotic Gradient (mOsm)** | **Sample History** | **Mean Particle Size* (nm)** |
|---|---|---|---|---|
| 0.9 | 5 | 146 | never frozen control | 1.14.3 ± 1.5 |
| 0.9 | 5 | 146 | frozen | 134.3 ± 2.1 |
| 0.6 | 5 | 43 | frozen | 138.3 ± 1.5 |
| 0.3 | 5 | -59 | frozen | 143.7 ± 1.2 |

| | | | | |
|---|---|---|---|---|
| *Mean particle size is the standard deviation of three measurements on a single sample. | | | | |

The data shown in Table 2 reveal that the extent of freeze/thaw damage, as determined by the increase in particle size after freezing, is influenced by the osmotic gradient across the liposome lipid bilayer. The liposome formulation having an external medium of 5% sucrose and 0.9% NaCl (osmotic gradient = 146 mOsm), was the least susceptible to freeze/thaw damage, as evidenced by the smallest increase in particle size of the three liposome formulations. The liposome formulation with a reversed osmotic gradient, that is the formulation with 5% sucrose and 0.3% NaCl in the external medium (osmotic gradient = -59 mOsm), had the largest increase in particle size upon freezing and thawing.

Example 3 describes a qualitative test on 24 liposome formulations to further assess the importance of an osmotic gradient on the susceptibility of a liposome formulation to freeze/thaw damage. In this example, placebo liposome formulations having the above lipid composition (HSPC, cholesterol and mPEG-DSPE) were prepared. The internal liposome medium was either 0.9% or 1.8% (w/v) NaCl. The external aqueous medium contained: (i) 10 millimolar (mM) histidine, (ii) 0%, 5% or 10% (w/v) sucrose, and (iii) a NaCl concentration equal to the internal NaCl concentration multiplied by 0.5, 1, 1.5 or 2.0. The internal and external media of each formulation are set forth in Table 3.

**Table 3**

| **Sample #** | **Internal [NaCl] (%; w/v)** | **External [NaCl] (%; w/v)** | **External [sucrose] (%; w/v)** | **External Osmolarity (mOsm)** | **Osmotic Gradient (mOsm)** | **Visual Appearance after Freezing**¹ |
|---|---|---|---|---|---|---|
| 1 | 0.9 | 0.45 | 0 | 164 | -144 | particles |
| 2 | 0.9 | 0.45 | 5 | 310 | 2 | turbid |
| 3 | 0.9 | 0.45 | 10 | 456 | 148 | turbid |
| 4 | 0.9 | 0.9 | 0 | 318 | 10 | turbid |
| 5 | 0.9 | 0.9 | 5 | 464 | 156 | turbid |
| 6 | 0.9 | 0.9 | 10 | 610 | 302 | nc |
| 7 | 0.9 | 1.35 | 0 | 472 | 164 | nc |
| 8 | 0.9 | 1.35 | 5 | 618 | 310 | nc |
| 9 | 0.9 | 1.35 | 10 | 764 | 456 | nc |
| 10 | 0.9 | 1.8 | 0 | 626 | 318 | nc |
| 11 | 0.9 | 1.8 | 5 | 772 | 464 | nc |
| 12 | 0.9 | 1.8 | 10 | 918 | 610 | nc |
| 13 | 1.8 | 0.9 | 0 | 318 | -298 | particles |
| 14 | 1.8 | 0.9 | 5 | 46.4 | -152 | particles |
| 15 | 1.8 | 0.9 | 10 | 610 | -6 | turbid |
| 16 | 1.8 | 1.8 | 0 | 626 | 10 | turbid |
| 17 | 1.8 | 1.8 | 5 | 772 | 156 | turbid |
| 18 | 1.8 | 1.8 | 10 | 918 | 302 | nc |
| 19 | 1.8 | 2.7 | 0 | 934 | 318 | nc |
| 20 | 1.8 | 2.7 | 5 | 1080 | 464 | nc |
| 21 | 1.8 | 2.7 | 10 | 1226 | 610 | nc |
| 22 | 1.8 | 3.6 | 0 | 1242 | 626 | nc |
| 23 | 1.8 | 3.6 | 5 | 1388 | 772 | nc |
| 24 | 1.8 | 3.6 | 10 | 1534 | 918 | nc |

| | | | | | | |
|---|---|---|---|---|---|---|
| ¹*nc* = no significant change in visual appearance compared to unfrozen control; *particles* = visual presence of macroscopic particles; *turbid* = sample was less translucent than unfrozen control. | | | | | | |

The samples shown in Table 3 were frozen at -20°C for 3 hours and subsequently thawed to room temperature. Each sample was visually inspected for macroscopic evidence of freezing damage, such as the presence of macroscopic particles, and for clarity of the suspension. The samples having a hypo-osmotic gradient (sample numbers 1, and 13-14 in Table 3, where the external osmolarity is less than the internal osmolarity), were visually heterogeneous with macroscopic particles visible after freezing and thawing. In contrast, liposome formulations having a hyper-osmotic gradient (sample numbers 3, 5-12, and 17-24 in Table 3), were visually similar to the unfrozen control samples. Nearly iso-osmotic samples (sample numbers 2, 4, and 15-16 in Table 3), had an intermediate appearance with not as many particles present as compared with the hypo-osmotic samples, but not as visually clear as the control. It is worth noting that the nearly iso-osmotic sample (sample #16) having 1.8% internal and external NaCl, was intermediate in appearance, indicating that protection from freezing damage is not simply a result of an increase in the external NaCl concentration, but is also the result of a hyperosmotic gradient across the liposome lipid bilayer.

In another experiment performed in support of the invention, and described in Example 4, liposome formulations containing cisplatin were prepared. The liposomes were composed of HSPC, cholesterol, and mPEG-DSPE in a 51/44/5 molar ratio, respectively. Entrapped in the liposomes was cisplatin in a 0.9% (w/v) NaCl solution. The external bulk suspension medium was composed of 10 mM histidine, NaCl at a concentration of 0.9%, 1.8%, 2.7%, or 3.6% (w/v), and sucrose at a concentration of 5%, 10%, 15%, or 20% (w/v). The exemplary formulations are set forth in Table 4. The liposome samples were frozen at -20°C for 6.8 days (approximately 118 hours), and then thawed by resting at room temperature. The mean particle size of the liposomes in each sample was determined as a measurement of the extent of freeze damage. The results are shown in Table 4.

**Table 4**

| **External [sucrose] (% w/v)** | **External [NaCl] (%; w/v)** | **Osmotic Gradient (mOsm)** | **Mean Particle Size (nm); never frozen** | **Mean Particle Size (nm); frozen at -20 °C 6.8 days** | **Increase in Mean Particle Size due to freezing*** (nm) |
|---|---|---|---|---|---|
| 5 | 0.9 | 156 | 112.3 ± 2.5 | 1450 ± 870 | 1340 ± 870 |
| 5 | 1.8 | 464 | 106.3 ± 0.6 | 133.7 ± 0.6 | 27.4 ± 1.2 |
| 5 | 2.7 | 772 | 104.3 ± 1.5 | 109.7 ± 1.5 | 5.4 ± 3.0 |
| 5 | 3.6 | 1080 | 103.3 ± 2.1 | 104.7 ± 2.3 | 1.4 ± 4.4 |
| 10 | 0.9 | 302 | 110.7 ± 0.6 | 169.3 ± 1.5 | 58.6 ± 2.1 |
| 10 | 1.8 | 610 | 104.7 ± 1.2 | 138.3 ± 1.5 | 33.6 ± 2.7 |
| 10 | 2.7 | 918 | 102.7 ± 1.5 | 115.7 ± 2.3 | 13.0 ± 3.8 |
| 10 | 3.6 | 1226 | 104.3 ± 1.2 | 110.7 ± 0.6 | 6.4 ± 1.8 |
| 15 | 0.9 | 448 | 108.3 ± 1.2 | 169.7 ± 0.6 | 61.4 ± 1.8 |
| 15 | 1.8 | 756 | 104.0 ± 1.0 | 132.0 ± 2.0 | 28.0 ± 3.0 |
| 15 | 2.7 | 1064 | 105.0 ± 1.0 | 120.7 ± 1.2 | 15.7 ± 2.2 |
| 15 | 3.6 | 1372 | 103.3 ± 2.1 | 111.0 ± 1.0 | 7.7 ± 3.1 |
| 20 | 0.9 | 594 | 104.3 ± 0.6 | 139.3 ± 1.5 | 35.0 ± 2.1 |
| 20 | 1.8 | 902 | 105.3±1.2 | 123.7 ± 2.5 | 18.4 ± 3.7 |
| 20 | 2.7 | 1210 | 104.0± 1.0 | 123.3 ± 1.5 | 19.3 ± 2.5 |
| 20 | 3.6 | 1518 | 103.7 ± 1.5 | 113.3 ± 1.5 | 9.6 ± 3.0 |

| | | | | | |
|---|---|---|---|---|---|
| *Mean particle size is the standard deviation of three measurements on a single sample. | | | | | |

As shown in Table 4, the increase in mean particle size upon a single freeze/thaw cycle is less when the external NaCl concentration is increased above that of isotonic, i.e., 0.9% (w/v), NaCl. At each concentration of sucrose, e.g. 5%, 10%, 15% and 20% (w/v), the higher the NaCl concentration, the better the liposome vesicles were protected from freeze/thaw damage. The external composition which provided the best protection from freezing damage was at 5% (w/v) sucrose, and 3.6% (w/v) NaCl, wherein the particle had no statistical increase in particle size.

As evidenced by the data shown in the fourth column of Table 4, even in the absence of freezing, the mean particle size decreased measurably with increasing external osmolarity. Experiments herein indicate that the decrease in size is not the result of a change in liposome shape. While liposome shrinkage is expected in the presence of a hyperosmotic gradient, due to the expulsion of water, a change in liposome shape should not persist after the 300-fold dilution with 0.9% (w/v) NaCl which occurs prior to the particle size measurement by dynamic light scattering. Experiments showed that diluting hypertonic formulations with 3.6% (w/v) NaCl or with distilled water, resulted in the same measured mean particle size as samples diluted with 0.9% (w/v) NaCl. This suggests that the decrease in measured particle size with increasing osmolarity is primarily due to a change in the aggregation state of the vesicles.

A surprising result shown in Table 4, is that increasing the external sucrose concentration did not significantly decrease the extent of particle size growth upon freezing and thawing, particularly when the external NaCl concentration was greater than or equal to 1.8% (w/v). When the external NaCl concentration was 2.7% or 3.6%, the data suggest that a lower external sucrose concentration may offer the best protection from freezing damage for this particular liposome composition. This point was further investigated by preparing liposome formulations having the same composition as set forth in Example 4, except that the external sucrose concentration was either 1%, 2%, 3%, 4%, or 5% (w/v). The samples were frozen at -20°C for 3.9 days (approximately 94 hours), and thawed to room temperature, and the extent of freezing damage was determined by measuring mean particle size before and after freezing. Results are shown in Table 5.

**Table 5**

| **External [sucrose] (% w/v)** | **External [NaCl] (%; w/v)** | **Osmotic Gradient (mOsm)** | **Mean Particle Size (nm); never frozen** | **Mean Particle Size (nm); frozen at -20 °C 3.9 days** | **Increase in Mean Particle Size due to freezing* (nm)** |
|---|---|---|---|---|---|
| 1 | 1.8 | 347 | 109.7 ± 0.6 | 225.0 ± 2.6 | 115.3 ± 3.2 |
| 1 | 2.7 | 655 | 106.7 ± 1.2 | 114.7 ± 0.6 | 8.0 ± 1.8 |
| 1 | 3.6 | 963 | 103.7 ± 1.2 | 106.0 ± 0.0 | 2.3 ± 1.2 |
| 2 | 1.8 | 376 | 108.7 ± 2.3 | 199.0 ± 2.6 | 90.3 ± 4.9 |
| 2 | 2.7 | 684 | 107.0 ± 1.0 | 112.7 ± 1.2 | 5.7 ± 2.2 |
| 2 | 3.6 | 992 | 104.3 ± 0.6 | 104.7 ± 0.6 | 0.4 ± 1.2 |
| 3 | 1.8 | 406 | 109.0 ± 0.0 | 174.3 ± 1.2 | 65.3 ± 1.2 |
| 3 | 2.7 | 714 | 107.0 ± 0.0 | 108.0 ± 1.0 | 1.0 ± 1.0 |
| 3 | 3.6 | 1022 | 105.0 ± 0.0 | 104.0 ± 1.0 | -1.0 ± 1.0 |
| 4 | 1.8 | 435 | 111.0 ± 1.0 | 160.3 ± 0.6 | 49.3 ± 1.6 |
| 4 | 2.7 | 743 | 104.7 ± 1.5 | 108.3 ± 0.6 | 3.6 ± 2.1 |
| 4 | 3.6 | 1051 | 105.3 ± 0.6 | 105.7 ± 0.6 | 0.4 ± 1.2 |
| 5 | 1.8 | 464 | 110.7 ± 1.2 | 142.7 ± 1.5 | 32.0 ± 2.7 |
| 5 | 2.7 | 772 | 106.0 ± 1.7 | 106.7 ± 1.2 | 0.7 ± 2.9 |
| 5 | 3.6 | 1080 | 104.0 ± 1.0 | 105.0 ± 1.0 | 1.0 ± 2.0 |

| | | | | | |
|---|---|---|---|---|---|
| *Mean particle size is the standard deviation of three measurements on a single sample. | | | | | |

The results shown in Table 5 are consistent with the results shown in Table 4, showing that at high external NaCl concentrations, the optimum external sucrose concentration for cryoprotection is relatively low for this particular liposome formulation.

The experiments described above focus primarily on particle size growth upon freezing and subsequent thawing, as evidenced by the extent of freeze/thaw damage. Additional experiments, described herein, relate to the loss of an entrapped therapeutic agent to assess the extent of freeze/thaw damage. In this experiment, some of the samples tested for the data of Table 5 were further analyzed for percent of drug encapsulation before and after freezing. The percent of entrapped cisplatin was measured by flame atomic absorption spectroscopy as described in the methods section below. The results are set forth in Table 6.

**Table 6**

| **External [sucrose] (%; w/v)** | **External [NaCl] (%; w/v)** | **Sample History (frozen or never frozen)** | **Increase in Mean Particle Size Due to Freezing (nm); from Table 5*** | **% Encapsulation** |
|---|---|---|---|---|
| 1 | 1.8 | never frozen control | not applicable | 100 |
| 1 | 1.8 | frozen | 115.3 ± 3.2 | 91 |
| 3 | 1.8 | frozen | 65.3 ± 1.2 | 91 |
| 3 | 2.7 | frozen | 1.0 ± 1.0 | 98 |
| 3 | 3.6 | frozen | -1.0 ± 1.0 | 98 |

| | | | | |
|---|---|---|---|---|
| *Mean particle size is the standard deviation of three measurements on a single sample. | | | | |

The correlation between percent encapsulation of cisplatin and mean particle size growth is evident. For example, release of cisplatin upon freezing and subsequent thawing was found to occur when there was particle size growth, i.e., increase as measured by dynamic light scattering (DLS).

To ensure that a small number of relatively large particles were not formed during freezing, turbidity measurements at 650 nm were performed (as detailed in the methods section below). Turbidity measurements are generally more sensitive than dynamic light scattering to small portions of large particles in a sample. Liposomes were prepared as described in Example 4, but with the external sucrose concentration held constant at 3% (w/v). The range of external NaCl concentrations is set forth in Table 7. The samples were frozen at -20°C for 3.9 days (approximately 93 hours), and thawed by resting at room temperature. The samples were then analyzed by DLS and turbidity determination, and compared to a control, i.e., a sample that was never frozen. The results are shown in Table 7 below.

**Table 7**

| **External [NaCl] (% w/v)** | **OD**_{**650 nm**} **(never frozen)** | **OD**_{**650 nm**} **(previously frozen)** | **Relative Change in Turbidity Due to Freezing and Thawing** | **Increase in Mean Particle Size Due to Freezing and Thawing (nm)*** |
|---|---|---|---|---|
| 1.8 | 0.603 | 2.200 | 3.65 | 58.7 ± 7.4 |
| 2.1 | 0.621 | 1.174 | 1.89 | 29.0 ± 3.0 |
| 2.4 | 0.647 | 0.738 | 1.14 | 6.4 ± 1.2 |
| 2.7 | 0.667 | 0.711 | 1.07 | 5.3 ± 2.5 |
| 3.0 | 0.667 | 0.676 | 1.01 | 2.0 ± 1.2 |
| 3.3 | 0.675 | 0.674 | 1.00 | 1.3 ± 2.6 |
| 3.6 | 0.675 | 0.687 | 1.02 | 3.0 ± 1.2 |

| | | | | |
|---|---|---|---|---|
| *Mean particle size is the standard deviation of three measurements on a single sample. | | | | |

The relative change in turbidity caused by one freeze/thaw cycle is determined by the ratio of turbidity_{(frozen and thawed)} to turbidity_{(never frozen)}. Comparing the last two columns of Table 7, the correlation between mean particle size growth and relative change in turbidity due to protection from freezing and thawing is evident. The data suggest the resistance to freeze/thaw damage provided by the presence of an osmotic gradient across the liposome bilayer is good, particularly when the external sucrose concentration is between about 1% to about 10%, more preferably between about 1% and about 5%, and most preferably between about 2% to about 4%, in the presence of greater than about 3% (w/v) NaCl.

In particular, the liposomal composition containing cisplatin used in the experiments described above, is protected from freeze/thaw damage in the presence of an external medium having between about 2.7% to about 3.6% (w/v) NaCl, and between about 2% to about 5% sucrose.

In an additional experiment, liposome formulations containing various amounts of a cryoprotectant and salt, were exposed to different freeze/thaw conditions. Liposomes were prepared from HPSC, mPEG-DSPE, and cholesterol as previously described. Cisplatin in a 0.9% (w/v) NaCl solution was entrapped in the liposomes and the external medium was adjusted to contain 3.3% (w/v) NaCl and 3% (w/v) sucrose. Samples were prepared in triplicate and were frozen by several methods, including: (i) dry ice/isoproponal bath for about 10 minutes, followed by incubation in a -70°C freezer; (ii) dry ice/isopropanol followed by incubation in a -20°C freezer; (iii) stored directly in a - 70°C freezer; (iv) stored directly in a -35°C freezer; and (v) stored directly in a - 20°C freezer. After 3.9 days, (approximately 94 hours), all of the samples were subsequently thawed. From each set of triplicates, one sample was thawed in room temperature water, one sample was thawed in room temperature air, and one sample was thawed in a 2-8°C refrigerator. All samples were assessed for freeze/thaw damage by measuring particle size by DLS. The only samples which exhibited measurable size growth were those samples frozen at -70°C, either directly, or after introducing to a dry ice/isopropanol bath followed by incubation in a -70°C freezer. The size growth was dependent on both the freezing and the thawing conditions, with the slower rates of freezing and thawing causing the most freeze/thaw damage, as shown below in Table 8.

**Table 8**

| **Freezing Conditions** | **Thawing Conditions** | **Sample**^{**1**} **History (Frozen or** Never Frozen) | **Mean Particle Size (nm)*** | **Size Growth (nm) after Freezing and Thawing** |
|---|---|---|---|---|
| N.A² | N.A. | never frozen control | 89.5 ± 0.7 | N.A. |
| DI/IPA, -70 | RT water | frozen | 90.3 ± 1.4 | 0.8 ± 2.1 |
| DI/IPA, -70 | RT air | frozen | 92.4 ± 1.9 | 2.9 ± 2.6 |
| DI/IPA, -70 | 2-8 °C air | frozen | 97.0 ± 1.8 | 7.5 ± 2.5 |
| -70 °C directly | RT water | frozen | 91.1 ± 1.8 | 1.6 ± 2.5 |
| - 70 °C directly | RT air | frozen | 97.3 ± 1.3 | 7.8 ± 2.0 |
| -70 °C directly | 2-8 °C air | frozen | 100.3 ± 0.6 | 10.8 ± 1.3 |

| | | | | |
|---|---|---|---|---|
| ¹All liposomes have osmotic gradient of 919 mOsm (0.9% internal NaCl; 3.3% external NaCl + 3% external sucrose + 10 mM external histidine; cisplatin is assumed to be predominantly precipitated.) | | | | |
| ²Abbreviations: N.A. = not applicable; DI/IPA, -70 = dry ice/isopropanol then placed in -70°C freezer; RT = room temperature. | | | | |
| *Mean particle size is the standard deviation of three measurements on a single sample. | | | | |

This experiment suggests that liposome compositions having an osmotic gradient are protected against freeze/thaw damage to temperatures of at least - 35°C.

The above experiments have demonstrated that an osmotic gradient across the liposome bilayer provides protection against freeze/thaw damage to the liposome compositions. However, it is also necessary that the osmotic gradient does not affect the stability of the liposome composition under non-freezing conditions. To assess the stability of the liposome compositions having an osmotic gradient, samples were placed in an accelerated stability test. Specifically, two lots of liposome compositions were prepared as described in Example 4. The liposome compositions contained HSPE, mPEG DSPE, and cholesterol with entrapped cisplatin. The external aqueous suspension medium was composed of 3.3% (w/v) NaCl, and 3% (w/v) sucrose Table 9 shows the components and concentrations for test Batches 1 and 2, and for the control formulation.

**Table 9**

| **Component** | **Concentration in mg/ml for Test Batch 1 and 2** | **Concentration in mg/ml for Control** |
|---|---|---|
| cisplatin | 1.0 | 1.0 |
| HSPC | 40.0 | 40.0 |
| cholesterol | 17.1 | 17.1 |
| mPEG-DSPE | 14.3 | 14.3 |
| sucrose | 25.5 | 0 |
| NaCl | 29.4 | 9 |
| histidine | 1.55 | 1.55 |

The two test batches and the control formulation were stored at various temperatures (as shown in Table 10) for up to 3.5 months. At intervals during this 3.5 month period, samples were removed for analysis by DLS to determine the percent of encapsulated cisplatin and mean particle size. The results are summarized in Table 10.

**Table 10**

| **batch #** | **Storage Temperature** | **Incubation Time** | **[Cisplatin] (mg/ml)** | **Percent Encapsulation** | **Mean Particle Size* (nm)** |
|---|---|---|---|---|---|
| 1 | N.A. | time zero | 0.897 ± 0.006 | 100.0 ± 0.0 | N.D. |
| 1 | +20 °C | 1 month | 0.900 ± 0.000 | 99.7 ± 0.6 | 98.5 ± 1.6 |
| 1 | +20 °C | 2 months | 0.877 ± 0.006 | 99.3 ± 0.6 | 98.4 ± 1.2 |
| 1 | +20 °C | 3 months | 0.877 ± 0.006 | 98.7 ± 0.6 | 97.7 ± 1.1 |
| 1 | +20 °C | 3.5 months | 0.907 ± 0.006 | N.D. | N.D. |
| 1 | 2-8 °C | 1 month | 0.91 | 100 | 104.0 ± 1.7 |
| 1 | 2-8 °C | 2 months | 0.89 | 100 | 100.0 ± 0.9 |
| 1 | 2-8 °C | 3 months | 0.93 | 100 | 100.2 ± 0.8 |
| 1 | -20 °C | 1 week | 0.89 | 94 | 102.3 ± 2.1 |
| 1 | -20 °C | 2 weeks | 0.89 | 91 | 110.3 ± 1.2 |
| 1 | -20 °C | 1 months | 0.90 | 88 | 108.3 ± 1.5 |
| 1 | -20 °C | 2 months | 0.87 | 87 | 110.7 ± 2.3 |
| 1 | -20 °C | 3 months | 0.89 | 84 | 111.7 ± 0.6 |
| 2, | N.A. | time zero | 0.930 ± 0.000 | 99 | N.D. |
| 2 | +20 °C | 1:3 months | 0.950 ± 0.000 | 99.0 ± 0.0 | 100.8 ± 2.1 |
| 2 | +20 °C | 2.3 months | 0.895 ± 0.005 | 97.0 ± 0.0 | 100.8 ± 0.8 |
| 2 | +20 °C | 3.3 months | 0.930 ± 0.010 | 96.3 ± 0.6 | 101.1 ± 0.8 |
| 2 | 2-8° | 1.3 months | 0.99 | 99 | 103.7 ± 1.2 |
| 2 | 2-8 ° | 2.3 months | 0.94 | 99 | 99.9 ± 1.2 |
| 2 | 2-8 ° | 3.3 months | 1.00 | 99 | 100.6 ± 1.6 |
| 2 | -20 °C | 2 weeks | 0.935 ± 0.005 | 95.5 ± 0.5 | 102.7 ± 1.2 |
| 2 | -20 °C | 1.3 months | 0.93 | 94 | 104.7 ± 0.6 |
| 2 | -20 °C | 2.3 months | 0.88 | 93 | 102.0 ± 1.0 |
| 2 | -20 °C | 3.3 months | 0.93 | 93 | 101.0 ± 1.0 |

| | | | | | |
|---|---|---|---|---|---|
| *Mean particle size is the standard deviation of three measurements on a single sample. | | | | | |

Table 10 illustrates, inter alia, that freezing batch #1 at -20°C for one week resulted in a 6% drop in the amount of encapsulated drug. In this sample, after 2 weeks of freezing, the percent of encapsulated drug lost was 9%. As shown, the mean particle size at these time points had only a slight increase. Infusion of a product exhibiting a 9% loss of the selected entrapped drug demonstrating little change in the particle size should not compromise efficacy, or patient safety, as a majority of the drug is entrapped in the liposomes. As shown, batch #2 exhibited even less leakage than bath #1.

The stability tests indicate that a liposome composition having an osmotic gradient across the lipid bilayer, wherein the outer liposome osmolarity is higher than the inner osmolarity, provides additional protection from accidental freezing damage during product shipment, with negligible effect on product stability.

### III. Liposome Components

### A. Lipids

In the studies discussed above employing an osmotic gradient, i.e., wherein the outer liposome osmolarity is higher than the inner osmolarity, to decrease liposome susceptibility to freezing damage, was demonstrated using liposomes composed of hydrogenated soy phosphatidylcholine (HSPC), cholesterol and mPEG-DSPE. It will be appreciated by one of skill in the art, however, that liposome compositions, composed of a variety of lipid compositions are contemplated. Liposomes are composed primarily of vesicle-forming lipids, i.e., lipids that can form spontaneously into bilayer vesicles in water, as exemplified by phospholipids. The vesicle-forming lipids of this type preferably have two hydrocarbon chains, typically acyl chains, and a head group, either polar or nonpolar. There are a variety of synthetic vesicle-forming lipids and naturally-occurring vesicle-forming lipids, including the phospholipids, such as phosphatidylcholine, phosphatidylethanolamine, phosphatidylglycerol, phosphatidylserine, phosphatidic acid, phosphatidylinositol, and sphingomyelin, wherein the two hydrocarbon chains are typically between about 12-22 carbon atoms in length, and have varying degrees of unsaturation. The above-described lipids and phospholipids whose acyl chains have varying degrees of unsaturation can be obtained commercially or prepared according to published methods. References for lipid synthesis include: (i) Mason et al., "A method for the synthesis of isomerically pure saturated mixed-chain phosphatidylcholines," Anal. Biochem., 113(1):96-101 (1981); (ii) Zalipsky, S. "Synthesis of an endgroup functionalized polyethylene glycol-lipid conjugate for preparation of polymer-grafter liposomes," Bioconjug. Chem., 4(4): 296-299 (1993).

The liposomes can also include a lipid that is stably incorporated into the liposome lipid bilayer, such as diacylglycerols, lyso-phospholipids, fatty acids, glycolipids, cerebrosides and sterols, such as cholesterol.

If desired, the liposome can also include a vesicle-forming lipid derivatized with a hydrophilic polymer, as described, for example, in U.S. Patent No. 5,013,556. Including a derivatized lipid in the liposome composition forms a surface coating of hydrophilic polymer chains around the liposome. The surface coating of hydrophilic polymer chains is effective to increase the *in vivo* blood circulation lifetime of the liposomes when compared to liposomes lacking such a coating.

Vesicle-forming lipids suitable for derivatization with a hydrophilic polymer include any of those lipids listed above, and, in particular phospholipids, such as distearoyl phosphatidylethanolamine (DSPE).

Hydrophilic polymers suitable for derivatization with a vesicle-forming lipid include polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxyethylacrylate, hydroxymethylcellulose, hydroxyethylcellulose, polyethyleneglycol, polyaspartamide and hydrophilic peptide sequences. The polymers may be employed as homopolymers, copolymers, and block or random copolymers.

A preferred hydrophilic polymer chain is polyethyleneglycol (PEG), preferably as a PEG chain having a molecular weight between about 500 daltons to about 10,000 daltons, more preferably between about 1,000 daltons to about 5,000 daltons. Methoxy or ethoxy-capped analogues of PEG are also preferred hydrophilic polymers, commercially available in a variety of polymer sizes, e.g., about 120 daltons to about 20,000 daltons.

Preparation of vesicle-forming lipids derivatized with hydrophilic polymers has been described, for example, in U.S. Patent No. 5,395,619. Preparation of liposomes including such derivatized lipids has also been described, which typically contain between about 1-20 mole percent of such a derivatized lipid included in the liposome composition.

### B. Entrapped Agent

The liposomes also typically include an entrapped agent, and/or drug, wherein "entrapped" is intended to include encapsulation of the agent and/or drug in the aqueous core and aqueous spaces of liposomes, in addition to entrapment of the agent and/or drug in the lipid bilayer(s) of the liposomes.

Agents and/or drugs contemplated for use in the liposome composition of the invention are widely varied, and include, for example, both therapeutic and diagnostic applications.

Therapeutic agents and/or drugs include natural and synthetic compounds having the following therapeutic activities: anti-arthritic, anti-arrhythmic, anti-bacterial, anticholinergic, anticoagulant, antidiuretic, antidote, antiepileptic, antifungal, anti-inflammatory, antimetabolic, antimigraine, antineoplastic, antiparasitic, antipyretic, antiseizure, antisera, antispasmodic, analgesic, anesthetic, beta-blocking, biological response modifying, bone metabolism regulating, cardiovascular, diuretic, enzymatic, fertility enhancing, growth-promoting, hemostatic, hormonal, hormonal suppressing, hypercalcemic alleviating, hypocalcemic alleviating, hypoglycemic alleviating, hyperglycemic alleviating, immunosuppressive, immunoenhancing, muscle relaxing, neurotransmitting, parasympathomimetic, sympathominetric plasma extending, plasma expanding, psychotropic, thrombolytic and vasodilating.

In a preferred embodiment, the entrapped agent is a "cytotoxic drug", that is, a drug having a deleterious or toxic effect on targeted cells. Exemplary cytotoxic agents include the anthracycline antibiotics such as doxorubicin, daunorubicin, epirubicin and idarubicin, and analogs of these, such as epirubidin and mitoxantrone; platinum compounds, such as cisplatin, carboplatin, ormaplatin, oxaliplatin, zeniplatin, enloplatin, lobaplatin, spiroplatin, ((-)-(R)-2-aminomethylpyrrolidine (1,1-cyclobutane dicarboxylato)platinum)(DWA2114R), (SP-4-3(R)-1,1-cyclobutanedicarboxylato(2-)-(2-methyl-1,4-butanediamine-N,N')platinum) (CI-973), nedaplatin (254-S) and (bis-acetato-ammine-dichloro-cyclohexylamineplatinum(IV)) (JM-216) (Weiss et al., Drugs, 46(3):360-377 (1993)); and vinca alkaloids, such as vincristine, vinblastine, vinleurosine, vinrodisine, vinorelbine (navelbine) and vindesine.

Another group of cytotoxic agents are the topoisomerase I inhibitors, such as camptothecin and its analogues, including SN-38 ((+)-(4S)-4,11-diethyl-4,9-dihydroxy-1H-pyrano[3',4':6,7]-indolizino[1,2-b]quinoline-3,14(4H,12H)-dione); 9-aminocamptothecin; 9-nitrocamptothecin, topotecan (hycamtin; 9-dimethyl-aminomethyl-10-hydroxycamptothecin); irinotecan (CPT-11; 7-ethyl-10-[4-(1-piperidino)-1-piperidino]-carbonyloxy-camptothecin), which is hydrolyzed *in vivo* to SN-38); 7-ethylcamptothecin and its derivatives (Sawada et al., Chem. Pharm. Bull., 41(2):310-313 (1993)); 7-chloromethyl-10,11-methylene-dioxy-camptothecin; and others (SN-22, Kunimoto et al., J. Pharmacobiodyn., 10(3):148-151 (1987); DX-8951f and GG-211 ((7-(4-methylpiperazino-methylene)-10,11-ethylenedioxy-20(S)-camptothecin)) (Rothenberg, Ann. Oncol., 8(9):837-855 (1997)), and 7-(2-(N-isopropylamino)ethyl)-(20S)-camptothecin (Chong Kun Dang Corp., Seoul Korea, CKD602).

In a preferred embodiment of the invention, the composition is used for protection of liposomes entrapping a therapeutic agent and/or drug, having a high ionic strength, such as cisplatin and its related analogues, recited above.

### C. Salt and Cryoprotectant

The external suspension medium of the liposome composition typically has a higher osmolarity than the osmolarity of the internal liposome medium. The increased osmolarity is provided primarily by the addition of a salt to the external medium. Any water soluble salt is suitable for this purpose. For example, salts selected from nitrate salts, sodium salts, potassium salts, ammonium salts, and sulfate salts are contemplated. In a preferred embodiment, the salt is NaCl or KCI.

The external medium also includes a cryoprotectant, generally defined above to include sugars, glycerol, and polyethyleneglycol. The sugar can be a disaccharide or a monosaccharide, and exemplar sugars include trehalose, maltose, sucrose, glucose, lactose, dextran, and aminoglycosides.

All publications, patents and patent documents are incorporated by reference herein, as though individually incorporated by reference. The invention has been described with reference to various specific and preferred embodiments and techniques. However, it should be understood that many variations and modifications may be made while remaining within the spirit and scope of the invention.

The following Examples are intended to illustrate but not limit the invention.

**Materials:** DSPE was purchased from Avanti Polar Lipids (Birmingham, AL) and methoxy-polyethyleneglycol-distearoylphosphatidylethanolamine (mPEG-DSPE, mPEG MW 2000 Daltons) was obtained from Sygena, Inc. (Cambridge, MA). Cholesterol was obtained from Slovay Pharmaceuticals (Veenedaal, The Netherlands). HSPC was made by Lipoid K.G. (Ludwigshafen, Germany). Cisplatin was obtained from W.C. Heraeus GmbH (Hanau, Germany).

### Methods:

### 1. Liposome Particle Size

Liposome particle size was determined by dynamic light scattering using a Coulter model N4MD (Coulter Corp, Miami, FL). Unless noted otherwise, each liposome composition was diluted about 300-fold with 0.9% (w/v) NaCl before measuring at DSL measurement with a light beam at varying angles (90°and 30°). Measurements were made in triplicate at 20°C.

### 2. Percent Encapsulation of Cisplatin

Liposomes were separated from free drug by gel exclusion chromatography, with fractions analyzed for total Pt content by flame atomic absorption spectroscopy using a Perkin-Elmer model 3110.

### 3. Turbidity

Liposomes were diluted 5-fold with 0.9% (w/v) NaCl before measuring optical density at 650nm (OD_{650 nm}) at room temperature, using 0.9% NaCl (w/v) as a blank. The relative change in turbidity caused by freezing and thawing is defined as the ratio of OD_{650 nm}(frozen and thawed)-to-OD_{650 nm}(never frozen).

### Example 1

### Cryoprotection in Presence of Osmotic Gradient

### A. Placebo Liposome Preparation: Internal Sucrose

The lipids HSPC, cholesterol and mPEG-DSPE in a molar ratio of 51/44/5 were added to dehydrated ethanol at 60-65°C to give 0.002 moles of lipid per ml ethanol and mixed until dissolved, approximately 1 hour. The dissolved lipids were added to a solution of 0.9% (w/v) NaCl and 5% (w/v) sucrose in sterile water to yield a total lipid concentration of approximately 126 mg/ml. Samples were diluted after dialysis to a total lipid concentration of about 100 nM.

### B. Placebo Liposome Preparation: No internal Sucrose

The lipids HSPC, cholesterol and mPEG-DSPE in a molar ratio of 51/44/5 were added to dehydrated ethanol at 60-65°C to give 0.002 moles of lipid per ml ethanol and mixed until dissolved, approximately 1 hour. The dissolved lipids were added to a solution of 0.9% (w/v) NaCl in sterile water to yield a total lipid concentration of approximately 126 mg/ml.

For both preparations described in A and B above, liposomes were hydrated at 60-65°C with mechanical stirring for approximately 1 hour before formation of unilamellar vesicles of approximately 120 nm diameter by extrusion at 60-65°C using polycarbonate membranes having 0.1 micron pore size. Ethanol was removed and the external aqueous medium was adjusted to 0.9% (w/v) NaCl and 5% (w/v) sucrose by dialysis.

### C. Freezing and Thawing Conditions

A 3 mL, room temperature aliquot of the liposome suspensions formed in A and B above were placed in a 10 mL clear glass serum vial. The vials were placed in a conventional -20°C freezer 2.7 days. The samples were then removed from the freezer and placed at room temperature to thaw.

### D. Analysis of Samples

The particle size of the liposomes in each of the formulations after thawing as well as control samples of each formulation (control meaning that the sample was not frozen) were determined by dynamic light scattering. The results are shown in Table 1.

### Example 2

### Liposome Formulations with Various NaCl External Concentrations

Liposomes were prepared by dissolving the lipids HSPC, cholesterol and mPEG-DSPE in a molar ratio of 51/44/5 in dehydrated ethanol at 60-65°C to give 0.002 moles of lipid per ml ethanol. The dissolved lipids were added to a 0.9% (w/v) NaCl to yield a total lipid concentration of approximately 126 mg/ml. The aqueous lipid solution was mixed to form liposomes. The liposomes were subsequently down-sized by extrusion. After dialysis, the total lipid was brought down to about 100 mM (prior to the 3-fold dilutions with 5% sucrose and 0.9%, 0.45%, or 0% NaCl, described below).

The external bulk medium was exchanged with a medium containing 0.9% (w/v) NaCl and 5% (w/v) sucrose by dialysis. The formulation was diluted 3-fold with aqueous solutions of 5% (w/v) sucrose and 0.9%, 0.45% or 0% (w/v) NaCl, to yield an external medium of 5% (w/v) sucrose and 0.9%, 0.6% or 0.3% (w/v) NaCl.

Samples of each formulation were frozen for 4.9 days at -20°C. The samples were thawed by resting at room temperature and then the particle size was determined by dynamic light scattering. The results are shown in Table 2.

### Example 3

### Liposome Preparations of Table 3

The liposome preparations set forth in Table 3 were prepared as described in Examples 1 and 2, with the following modifications:
(i) for half the sampls the aqueous phase used for hydration was 0.9% NaCl and
   for the other half it was 1.8% NaCl; and
(ii) the dilution after dialysis was done with histidine together with varying amounts of NaCl and sucrose to bring external [NaCl] and [sucrose] to the values shown in Table 3 and contained 0.5mM pyranine. Pyranine is a fluorescent probe that does not affect the results of the freeze/thaw experiment. All hydration media, in addition to NaCl contained 0.5mM pyranine.

### Example 4

### Liposomes with Entrapped Cisplatin

Sterile water was heated to 63-67°C in a TEFLON-lined pressure vessel and sodium chloride (0.9%) was added. Cisplatin was added at a concentration of 8.5 mg/ml and mixed until dissolved, approximately 15-25 minutes. 257.0 g PEG-DSPE, 719.4 g HSPC and 308.4 g cholesterol (molar ratio of 50.6/44.3/5.1) were added to 900 ml dehydrated ethanol at 60-65°C and mixed until dissolved, approximately 2 hours. The dissolved lipids were added to 7670 g of drug solution to give a total lipid concentration of approximately 150 mg/ml.

The warm lipid solution was rapidly added to the warm (63-67°C) drug solution, with mixing, to form a suspension of liposomes having heterogeneous sizes. The suspension was mixed for one hour at 63-67°C. The cisplatin concentration in the hydration mixture was 7.7 mg/ml and, at this stage, approximately 30% of the drug was encapsulated in the liposomes. 10% of the total solution volume was ethanol and the total lipid concentration was 150 mg lipid/ml.

The liposomes were sized to the desired mean particle diameter by controlled extrusion through polycarbonate filter cartridges housed in Teflon-lined stainless steel vessels. The liposome suspension was maintained at 63-65°C throughout the extrusion process, a period of 6-8 hours.

After sizing, the liposome suspension was cooled to 2-8°C overnight and then warmed to room temperature (20-25°C) prior to filtering through a 1.2 µm 142-mm Gelman Versapor filter (acrylic copolymer on a Nylon 66 support) to remove precipitated drug.

A 0.9% (w/v) aqueous solution of NaCl was prepared by dissolving the NaCl in sterile water. The pH of the solution was adjusted to approximately 5.5 with 2 N HCl or NaOH. The solution was filtered through a 0.22 µm Durapore filter.

The liposome suspension was diluted in approximately a 1:1 (v/v) ratio with the NaCl solution and diafiltered through a polysulfone hollow-fiber ultrafilter. Eight volume exchanges were performed against the NaCl solution to remove the ethanol and unencapsulated drug. The process fluid temperature was maintained at about 20-30°C. Total diafiltration time was approximately 4.5 hours.

The liposome suspension was then concentrated to approximately 1.2 mg cisplatin/ml by ultrafiltration. The post diafiltration process fluid was analyzed for cisplatin content by HPLC. The liposomes had an internal phase of 7.7 mg/ml cisplatin in 0.9% NaCl and an external phase of aqueous 0.9% NaCl.

Samples of the liposome formulation were placed into glass vials, and solid NaCl and sucrose were added to yield external NaCl concentrations of between 0.9-3.6% (w/v) and external sucrose concentrations of between about 1% to about 20% (w/v), assuming the volume of the external medium was 85% of the total volume before the addition of solids. Each sample was frozen at - 20°C for 6.8 days and then thawed by resting at room temperature. The mean particle size of the liposomes in each sample was determined as a measurement of extent of freezing damage. The results are shown in Table 4.

Although the invention has been described with respect to particular embodiments, it will be apparent to those skilled in the art that various changes and modifications can be made without departing from the invention.

## Claims

1. A liposome composition having protection from freeze/thaw damage comprising:
a suspension of liposomes wherein each liposome contains an entrapped aqueous medium having an inner osmolarity, the liposomes being suspended in an external medium comprising a water soluble salt and a cryoprotectant, said external medium having an outer liposome osmolarity higher than the inner liposome osmolarity thereby establishing a lower inside/higher outside osmotic gradient across each liposome.

2. A composition according to claim 1, wherein the external medium includes a salt selected from the group consisting of nitrate salts, sodium salts, potassium salts, ammonium salts and sulfate salts.

3. A composition according to claim 1 or 2, wherein the cryoprotectant is a disaccharide sugar, a monosaccharide sugar, or is selected from the group consisting of trehalose, maltose, sucrose, glucose, lactose, dextran, glycerol, polyethyleneglycol, and aminoglycosides.

4. A composition according to claim 2 or 3, wherein the salt is selected from sodium chloride (NaCl) and potassium chloride (KCl), or a combination thereof.

5. A composition according to claim 3 or 4, wherein the salt in the external medium is NaCl and the cryoprotectant is sucrose.

6. A composition according to any preceding claim, wherein the liposomes include a vesicle forming lipid derivatized with a hydrophilic polymer chain.

7. A composition according to any preceding claim, further comprising a therapeutic agent entrapped in the liposome.

8. A composition according to claim 7 wherein the therapeutic agent comprises a metal coordination compound.

9. A composition according to claim 8, wherein the metal coordination compound is cisplatin or a cisplatin analogue or derivative thereof.

10. A method for protecting a liposome composition against freeze/thaw damage comprising:
preparing a suspension of liposomes according to any preceding claim.

11. A method for improving freezing a suspension of liposomes comprising:
combining in the suspension of liposomes a cryoprotectant and a water soluble salt effective to establish an osmotic gradient across each liposome, the suspension medium having a higher osmolarity than the liposome interior.

12. A composition according to any one of claims 1 to 9 or a method according to claim 10 or 11, wherein the osmotic gradient is at least 100 mOsm.

## Patentansprüche

1. Liposomzusammensetzung mit Schutz vor Gefrier-/Auftau-Schaden, umfassend:
eine Liposomensuspension, worin jedes Liposom ein eingeschlossenes wässriges Medium mit einer inneren Osmolarität enthält, wobei die Liposome in einem äußeren Medium suspendiert sind; das ein wasserlösliches Satz und ein Kälteschutzmittel enthält, wobei das äußere Medium eine äußere Liposomosmolarität aufweist, die höher ist als die innere Liposomosmolarität, wodurch ein osmotischer niedrig/hoch-Gradient von innen nach außen über jedem Liposom aufgebaut wird.

2. Zusammensetzung nach Anspruch 1, worin das äußere Medium ein Salz umfasst, das ausgewählt ist aus der Gruppe, die aus Nitratsalzen, Natriumsalzen, Kaliumsalzen, Ammoniumsalzen und Sulfatsalzen besteht.

3. Zusammensetzung nach den Ansprüchen 1 oder 2, worin das Kälteschutzmittel ein Disaccharidzucker, ein Monosaccharidzucker, oder aus der Gruppe ausgewählt ist, die aus Trehalose, Maltose, Saccharose, Glucose; Lactose, Dextran, Glycerin, Polyethylenglycol und Aminoglycosiden besteht.

4. Zusammensetzung nach den Ansprüchen 2 oder 3, worin das Salz ausgewählt ist aus Natriumchlorid (NaCl) und Kaliumchlorid (KCl) oder einer Kombination davon.

5. Zusammensetzung nach den Ansprüchen 3 oder 4, worin das Salz im externen Medium NaCl und das Kälteschutzmittel Saccharose ist

6. Zusammensetzung nach einem der vorhergehenden Ansprüche, worin die Liposome ein vesikelbildendes Lipid umfassen, das mit einer hydrophilen Polymerkette derivatisiert ist.

7. Zusammensetzung nach einem der vorhergehenden Ansprüche, die weiterhin ein therapeutisches Mittel umfasst, das in den Liposomen eingeschlossen ist.

8. Zusammensetzung nach Anspruch 7, worin das therapeutische Mittel eine Metall-Koordinationsverbindung umfasst.

9. Zusammensetzung nach Anspruch 8, worin die Metall-Koordinationsverbindung Cisplatin oder ein Cisplatin-Analoges oder ein Derivat davon ist.

10. Verfahren zum Schutz einer Liposomzusammensetzung vor Gefrier/Auftau-Schaden, umfassend:
Herstellen einer Liposomensuspension nach einem der vorhergehenden Ansprüche.

11. Verfahren zum verbesserten Einfrieren einer Liposomensuspension, umfassend:
Kombinieren eines Kälteschutzmittels und eines wasserlöslichen Salzes, das wirksam ist, einen osmotischen Gradienten über jedem Liposom aufzubauen, in der Liposomensuspension, wobei das Suspensionsmedium eine höhere Osmolarität als das Liposomeninnere hat.

12. Zusammensetzung nach einem der Ansprüche 1 bis 9 oder Verfahren nach den Ansprüchen 10 oder 11, worin der osmotische Gradient mindestens 100 mOsm beträgt.

## Revendications

1. Composition de liposomes ayant une protection à l'encontre de l'endommagement dû à la congélation/décongélation, comprenant :
- une suspension de liposomes dans laquelle chaque liposome contient un milieu aqueux piégé ayant une osmolarité interne, les liposomes étant en suspension dans un milieu externe comprenant un sel soluble dans l'eau et un cryoprotecteur, ledit milieu externe ayant une osmolarité de liposome externe supérieure à l'osmolarité de liposome interne, établissant de cette façon un gradient osmotique inférieur à l'intérieur/supérieur à l'extérieur à travers chaque liposome.

2. Composition selon la revendication 1, dans laquelle le milieu externe comprend un sel choisi dans le groupe constitué par les sels nitrates, les sels de sodium, les sels de potassium,: les sels d'ammonium et les sels sulfates.

3. Composition selon l'une des revendications 1 ou 2, dans laquelle le cryoprotecteur est un sucre disaccharide, un sucre monosaccharide, ou est choisi dans le groupe constitué par le tréhalose, le maltose, le sucrose, le glucose, le lactose, le dextrane, le glycérol, le polyéthylèneglycol et les aminoglycosides.

4. Composition selon l'une des revendications 2 ou 3, dans laquelle le sel est choisi parmi le chlorure de sodium (NaCl) et le chlorure de potassium (KCl), ou une combinaison de ceux-ci.

5. Composition selon l'une des revendications 3 ou 4, dans laquelle le sel dans le milieu externe:est NaCl et le cryoprotecteur est le sucrose.

6. Composition selon l'une quelconque des revendications précédentes, dans laquelle les liposomes comprennent un lipide de formation de vésicules transformé en un dérivé par une chaîne de polymère hydrophile.

7. Composition selon l'une quelconque des revendications précédentes, comprenant en outre un agent thérapeutique piégé dans le liposome.

8. Composition selon la revendication 7, dans laquelle l'agent thérapeutique comprend un composé de coordination métallique.

9. Composition selon la revendication 8, dans laquelle le composé de coordination métallique est le cisplatine ou un analogue du cisplatine ou un dérivé de ceux-ci.

10. Procédé de protection d'une composition de liposomes à l'encontre de l' endommagement dû à la congélation/décongélation, comprenant l'opération consistant à :
- préparer une suspension de liposomes telle que définie à l'une quelconque des revendications précédentes.

11. Procédé pour améliorer la congélation d'une suspension de liposomes, comprenant l'opération consistant à :
- combiner dans la suspension de liposomes un cryoprotecteur et un sel soluble dans l'eau effectif pour établir un gradient osmotique à travers chaque liposome, le milieu de suspension ayant une osmolarité supérieure à l'intérieur des liposomes.

12. Composition selon l'une quelconque des revendications 1 à 9 ou procédé selon l'une des revendications 10 ou 11, dans laquelle ou dans lequel le gradient osmotique est d'au moins 100 mOsm.
